# EUROPEAN PATENT APPLICATION

(11) **EP 4 721 786 A1**
(43) Date of publication of application: **08.04.2026**
(21) Application number: 24306642.0
(22) Date of filing: 07.10.2024
(51) Int. Cl.: A61M 5/32

(54) **SAFETY DEVICE FOR A MEDICAL CONTAINER, INJECTION DEVICE INCLUDING THIS SAFETY DEVICE AND THIS MEDICAL CONTAINER, AND METHOD FOR ASSEMBLING THIS INJECTION DEVICE**

(71) Applicant: Becton Dickinson France, 38800 Le Pont-de-Claix (FR)
(72) Inventor: VALENTIN, Stéphane, 38470 L'Albenc (FR); MILLS, Freddy, 38650 Sinard (FR); CHANIOL, Henry-Gilles, 38120 Fontanil-Cornillon (FR); ALVAIN, Olivier, 38180 Seyssins (FR); CARREL, Franck, 38220 Saint Jean de Vaulx (FR)
(74) Representative: Germain Maureau

(57) **Abstract**

This safety device (1) includes: a body (2) extending along a longitudinal axis A and configured for receiving a medical container (6) provided with an injection needle (66), a plunger rod (68), and a barrel (60), the barrel (60) including an emboss (62); a needle guard (3) axially movable with respect to the body (2) between an initial position and a safety position distally located with respect to the initial position; biasing means for moving the needle guard (3) from the initial position to the safety position; a retainer (32), movable between a blocking position, and a release position in which the retainer (32) no longer prevents the biasing means from moving the needle guard (3) to the safety position. Tthe safety device (1) further includes: a spacer (5) arranged between the body (2) and the medical container (6) for compensating a gap the body (2) and the medical container (6); an axial slot (52) extending along the spacer (5) for receiving the emboss (62) of the medical container (6); first fasteners (55, 56) for axially fixing the medical container (6) to the spacer (5); and second fasteners (58, 574) for axially fixing the spacer (5) to the body (2).

## Description

The present invention relates to a safety device and to an injection device including this safety device equipping a medical container such as a syringe, and more specifically a cut flange or round flange by-pass syringe.

In this application, the distal end of a component or of a device is to be understood as meaning the end furthest from the user's hand and the proximal end is to be understood as meaning the end closest to the user's hand. Likewise, in this application, the "distal direction" is to be understood as meaning the direction away from the user's hand, and the "proximal direction" is to be understood as meaning the direction toward the user's hand.

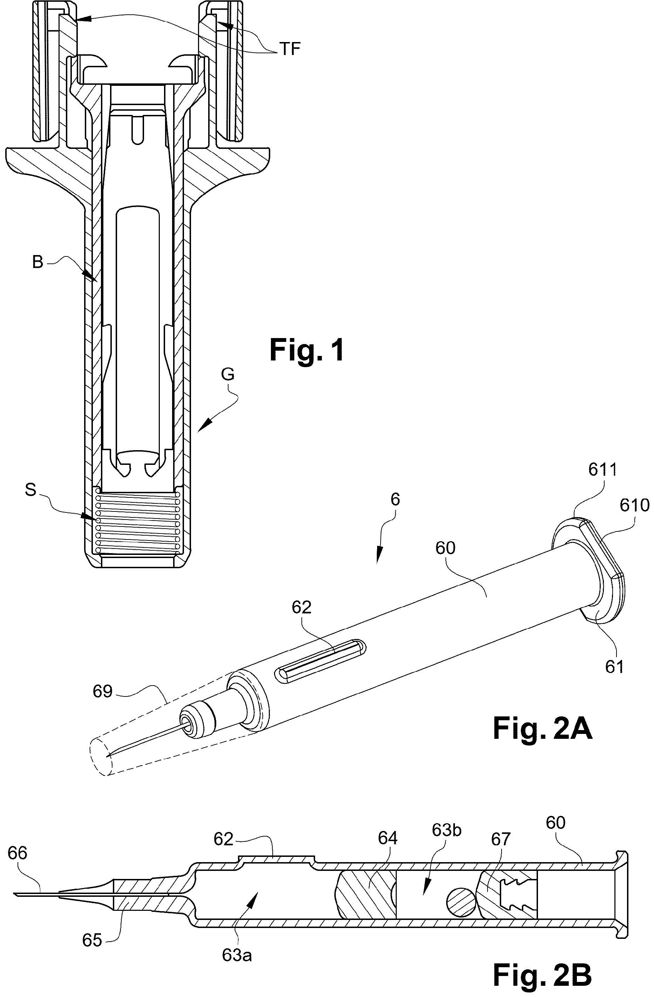

It is known to use safety devices, Figure 1A, configured for equipping a syringe. These safety devices include a body B for receiving the syringe, a guard G for covering a used needle, a plunger rod to dispense medicine from the syringe and a spring S for activation of the guard. The guard G and the body B are held together by two trigger fingers TF prior to use of the device.

Besides, it is known to use so-called by-pass syringes including a barrel and, on said barrel, an outer relief or emboss defining an inner cavity configured for allowing the mixing, or the subsequent delivery, of two different drugs contained in the by-pass syringe when a stopper, pushed by a plunger rod, reaches the inner cavity.

However, the known safety devices are usually configured for receiving purely cylindrical syringes and are thus not adapted to receive by-pass syringes. Indeed, the emboss of the by-pass syringe may be an issue for inserting the by-pass syringe into the safety device. Furthermore, the emboss may prevent the by-pass syringe from rotating within the safety device, which may prevent from reading a label affixed to the barrel of the syringe or visually inspecting the drugs contained within the by-pass syringe. The emboss also reduces the label dimension, since the label cannot be affixed onto the emboss. This may result in difficulties to include all the required data to be displayed on the label. If a radial gap separates the syringe from the body of the safety device in order to allow for proper insertion of the emboss, then the syringe alignment with the body will not be appropriate and this will have an impact in all device functions due to the clearances and uncontrolled mobility of the by-pass syringe within the body.

There is therefore a need for a safety device that alleviates some or all of the aforementioned drawbacks of the prior art, and more specifically for a safety device that is compatible with by-pass syringes.

In this context, an aspect of the invention is a safety device configured for protecting a user against needle stick injuries, the safety device including:
a body extending along a longitudinal axis A and configured for receiving a medical container provided with an injection needle, a plunger rod, and a barrel, the barrel including an emboss,
a needle guard axially movable with respect to the body between an initial position and a safety position distally located with respect to the initial position,
biasing means for moving the needle guard from the initial position to the safety position,
a retainer, movable between a blocking position in which the retainer blocks the needle guard in the initial position against the action of the biasing means, and a release position in which the retainer no longer prevents the biasing means from moving the needle guard to the safety position, movement of the retainer from the blocking position to the release position being caused by the plunger rod at the end of injection, wherein the safety device further includes
a spacer arranged between the body and the medical container for compensating a gap the body and the medical container,
an axial slot extending along the spacer for receiving the emboss of the medical container.
first fasteners for axially fixing the medical container to the spacer, and
second fasteners for axially fixing the spacer to the body.

The safety device of the invention, by means of the spacer, is therefore adapted to receive a by-pass syringe.

The device of the invention may further include some or all of the features listed below.

The spacer being configured such that the emboss does not radially outwardly protrude outside the axial slot, so that the emboss cannot interfere with the body. The axial slot has an opened proximal end to allow for insertion of the medical container within the spacer.

In an embodiment, the first fasteners include snap-fit elements configured for blocking axial movement of the medical container relative to the spacer in a proximal direction. The snap-fit elements accordingly prevent disassembly of the medical container from the spacer.

The medical container may be axially fixed to the spacer. That is, the medical container is not axially fixed to the body, but to the spacer. The spacer may be axially fixed to the body.

In an embodiment, the snap-fit elements are wholly positioned distal to a transversal plane including a distal blocking surface of the retainers. Thus, the snap-fit elements cannot hinder the abutment between the plunger rod and the retainers at the end of injection. The snap-fit elements do not extend in the proximal direction beyond a proximal abutment surface of the body or the distal blocking surface of the retainers.

In an embodiment, the snap-fit elements are configured for axially abutting against a proximal side of a flange of the medical container. The spacer may include a proximal abutment surface configured for stopping insertion of the medical container within the spacer. Thus, the proximal abutment surface blocks distal movement of the medical container relative to the spacer. The snap-fit elements may include a distal blocking surface configured for abutting against the body so as to stop insertion of the spacer within the body. Each of the snap-fit elements is circumferentially arranged between two blocking elements of the body. The blocking elements define an orthoradial abutment surface configured for orthoradially abutting against the snap-fit member to prevent the spacer from rotating relative to the body. The snap-fit member may include two orthoradial protrusions, each orthoradial protrusion extending below the blocking elements of the body, such that the orthoradial protrusions can proximally abut against the blocking elements of the body to prevent removal of the spacer from the body.

In an embodiment, the snap-fit elements include a radial abutment surface configured for radially abutting against an inner surface of the body to prevent radial outward movement of the snap-fit elements when the spacer is arranged within the body.

In an embodiment, the radial abutment surface is delimited by one or more axial ribs protruding from an outer surface of the snap-fit elements.

In an embodiment, a distal end of the axial slot opens on a window configured for receiving the emboss of the medical container and for allowing rotation of the medical container with respect to the spacer. The window may be configured for entirely receiving the emboss. The window may define a 120° opening around the longitudinal axis A. In an alternative embodiment, the axial slot has a closed distal end. Thus, the spacer is devoid of the window and the medical container cannot rotate with respect to the spacer.

In an embodiment, the second fasteners include a proximal abutment surface arranged on a radial outward protrusion for axially abutting against the body to prevent removal of the spacer from the body. The spacer may include two radial outward protrusions arranged on both sides of the axial slot. The radial outward protrusions may be arranged at 90° from the snap-fit elements.

In an embodiment, the radial outward protrusion are radially and/or axially distant from the needle guard to avoid interference between the spacer and the needle guard. Thus, the spacer does not hinder deployment of the needle guard after injection.

In an embodiment, the spacer includes one or more breakable bridges connecting two opposite sides of the axial slot of the spacer before insertion of the medical container within the spacer, the breakable bridges being configured to break upon radial outward deformation of the spacer when the medical container is inserted within the spacer. The snap-fit elements may include a radially outwardly deformable arm. In an alternative embodiment, the spacer is devoid of such breakable bridges. The snap-fit elements may be rigid, and thus devoid of a radially outwardly deformable arm.

Possibly, the spacer includes one or more deformable ties configured for limiting radial outward deformation of the spacer when the medical container is inserted within the spacer.

The ties allow for maintaining stiffness of the spacer by limiting its radial outward deformation. Unlike the breakable bridges, the ties are configured not to break, but to deform, thereby easing passage of the emboss and avoiding generation of small plastic particles.

According to another aspect, the invention relates to a drug delivery device including a medical container and the above-described safety device.

In an embodiment, the medical container may be a by-pass syringe.

In an embodiment, the medical container has a cut flange. A cut flange includes two parallel edges. Cut flange syringes differ from round flange syringes which have a 360° circular flange. In another embodiment, the medical container may have a round flange. The round flange has a 360° circular peripheral edge.

According to another aspect, the invention relates to a method for assembling the aforementioned injection device, the method comprises a step of assembling the safety device without the spacer to obtain a first sub-assembly; a step of assembling the medical container to the spacer to obtain a second sub-assembly; and a step of inserting the second sub-assembly into the first sub-assembly.

The invention and the advantages arising therefrom will clearly emerge from the detailed description that is given below with reference to the appended drawings as follows :
- Figure 1A is a cross-section view of a prior art safety device,
- Figure 2A is a perspective view of a by-pass syringe of an injection device according to an embodiment of the invention,
- Figure 2B is a cross-section of a by-pass syringe of an injection device according to an embodiment of the invention,
- Figure 3A is an exploded view of an injection device including a safety device according to an embodiment of the invention,
- Figure 3B is a perspective view of an injection device including a safety device according to an embodiment of the invention,
- Figure 4A is a perspective view of a spacer of a safety device according to an embodiment of the invention,
- Figure 4B is a partial view of the spacer of Figure 4A,
- Figure 5 is a perspective view of a spacer of a safety device according to an embodiment of the invention,
- Figure 6 is a perspective view of a spacer of a safety device according to an embodiment of the invention,
- Figure 7 is a perspective view of an injection device according to an embodiment of the invention, the needle guard being shown by transparency,
- Figure 8A is a partial cross-section view of the injection device of Figure 7,
- Figure 8B is a partial cross-section view of the injection device of Figure 7, at 90° from Figure 8A,
- Figure 9 illustrates assembly of an injection device according to an embodiment of the invention
- Figure 10A is a perspective view of a spacer of a safety device according to an embodiment of the invention,
- Figure 10B is a perspective view of a spacer of a safety device according to an embodiment of the invention.

The different features of the embodiments can be used in combination with and used with other embodiments as long as the combined parts are not inconsistent with or interfere with the operation of the device and assembly. This invention is not limited in its application to the details of construction and the arrangement of components set forth in the following description or illustrated in the drawings. The embodiments herein are capable of being modified, practiced or carried out in various ways. The phraseology and terminology used herein is for the purpose of description and should not be regarded as limiting. The use of "including," "comprising," or "having" and variations thereof herein is to encompass the items listed thereafter and equivalents thereof as well as additional items. Unless limited otherwise, the terms "connected," "coupled," and "mounted," and variations thereof herein are used broadly and encompass direct and indirect connections, couplings, and mountings. In addition, the terms "connected" and "coupled" and variations thereof are not limited to physical or mechanical connections or couplings. In contrast, when an element is referred to as being "directly connected" or "directly coupled" to another element, there are no intervening elements present. Unless otherwise defined, all terms (including technical and scientific terms) used herein have the same meaning as commonly understood by one of ordinary skill in the art to which this disclosure belongs. It will be further understood that terms used herein should be interpreted as having a meaning consistent with their meaning in the context of this specification and the relevant art and will not be interpreted in an idealized or overly formal sense unless expressly so defined herein. Further, terms such as distal, proximal, up, down, bottom, and top are relative, and are to aid illustration, but are not limiting. Relative terms such as "below" or "above" or "upper" or "lower" or "horizontal" or "vertical" may be used herein to describe a relationship of one element to another element as illustrated in the Figures. It will be understood that these terms and those discussed above are intended to encompass different orientations of the device in addition to the orientation depicted in the Figures. The embodiments are not intended to be mutually exclusive so that the features of one embodiment can be combined with other embodiments as long as they do not contradict each other. Terms of degree, such as "substantially", "about" and "approximately" are understood by those skilled in the art to refer to reasonable ranges around and including the given value and ranges outside the given value, for example, general tolerances associated with manufacturing, assembly, and use of the embodiments. The term "substantially" when referring to a structure or characteristic includes the characteristic that is mostly or entirely present in the structure. As used herein, the singular forms "a," "an," and "the" are intended to include the plural forms as well, unless the context clearly indicates otherwise. As used herein, the term "and/or" includes any and all combinations of one or more of the associated listed items. It will be understood that, although the terms first, second, etc., may be used herein to describe various elements, these elements should not be limited by these terms. These terms are only used to distinguish one element from another. For example, a first element could be termed a second element, and, similarly, a second element could be termed a first element without departing from the scope of the present disclosure. For simplification, the parts or elements of one embodiment which are found identically or similarly in the other embodiment will be identified using the same numerical references and will not be described again.

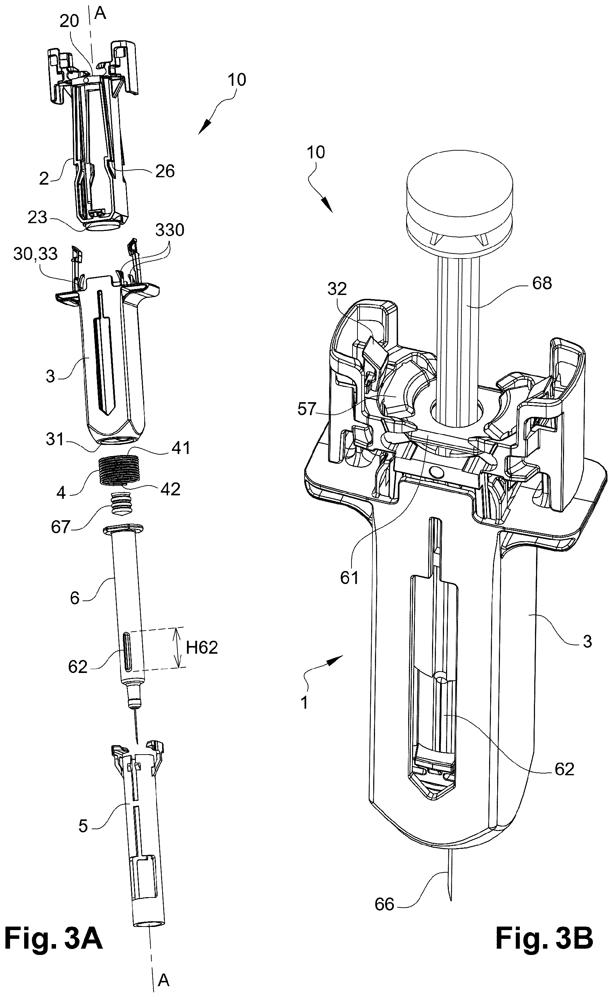

With reference to Figures 3A-3B is shown an injection device 10 according to an embodiment of the invention. The injection device 10 is configured for safely delivring a medical product to a patient, and includes a safety device 1 configured for receiving a medical container 6 and for protecting a user against needle stick injuries after injection.

The safety device 1 may include a body 2, a needle guard 3, a safety spring 4 acting as biasing means for moving the needle guard 3 from the initial position to the safety position at the end of injection, and a spacer 5.

The injection device 10 further includes a medical container 6, such as a prefillable or prefilled syringe. The syringe is a by-pass syringe, as shown in Figure 2B, i.e. is configured for sequential delivery or for mixing of two different drugs contained within the syringe. The medical container 6 includes a tubular barrel 60 having an emboss 62, the barrel 60 defining two reservoirs 63a, 63b each configured for containing a medical product. The two reservoirs are separated by a first stopper 64 configured for pushing the first medical product outside the barrel 60 via an elongated distal tip 65 to which is affixed an injection needle 66. A second stopper 67 is arranged within the barrel 60 for pushing the second medical product outside the barrel 60 via the emboss 62 and then the distal tip 65 and the injection needle 66. The second stopper 67 is moved by a plunger rod 68, Figure 3B. Prior to use of the injection device 10, the injection needle 66 is sealed and protected by a removable needle shield 69, Figure 2A, removably attached to the distal tip 65. At its proximal end, the barrel 60 may include a radial flange 61 which may be a cut flange 61, i.e. a flange 61 including two parallel straight edges 610, which may be connected by two circular portions 611. The barrel 60 may include or may be made of a glass or a plastic material. In another embodiment (not shown), the radial flange 61 may be a round flange, i.e. having a circular shape.

The body 2 extends along a longitudinal axis A and defines a proximal opening 20 configured for receiving the spacer 5 together with the medical container 6. The body 2 may include blocking members 21 in the form of inwardly radially extending lugs. The body 2 may further include a proximal abutment surface 22 configured for axially abutting against trigger fingers 32 of the needle guard 3 in orter to retain the needle guard 3 in the initial position before completion of injection.

The needle guard 3 extends around the body 2. The needle guard 3 includes a proximal opening 30 for receiving the body 2, a distal end 31, and two retainers in the form of trigger fingers 32 for retaining the needle guard 3 in the initial position. The trigger fingers 32 include a distal abutment surface 320 configured for axially abutting against the proximal abutment surface 22 of the body 2 to retain the needle guard 3 in the initial position. The trigger fingers 32 also include a camming surface 321 arranged on the axial path of the plunger rod 68 such that the plunger rod 68 abuts against the camming surface 321 at the end of injection, thereby radially outwardly deforming the trigger fingers 32 until the distal abutment surface 320 of the trigger fingers 32 moves out of contact from the proximal abutment surface 22 of the body 2, which releases the needle guard 3.

The needle guard 3 is axially movable with respect to the body 2 between an initial position, Figure 3B, and a safety position (not shown), distally located with respect to the initial position, in which the needle guard 3 exends over the injection needle 66 to protect the user against needle stick injuries. Distal movement from the initial position to the safety position is caused by the safety spring 4 and may be guided by the body 2. The safety spring 4 has a proximal end 41 seating against a distal end 23 of the body 2, and an opposite distal end 42 seating against a proximal seat 310 of the distal end 31 of the needle guard 3. The needle guard 3 may be rotationally fixed with respect to the body 2. Locking means, such as locking legs 330 arranged at a proximal end 33 of the needle guard 3, Figure 3A, for engaging a recess 24 of the body 2, may be provided to prevent the needle guard 3 in the safety position from moving back in the proximal direction. The needle guard 3, the trigger fingers 32 and the locking legs 330 may be made of a single piece.

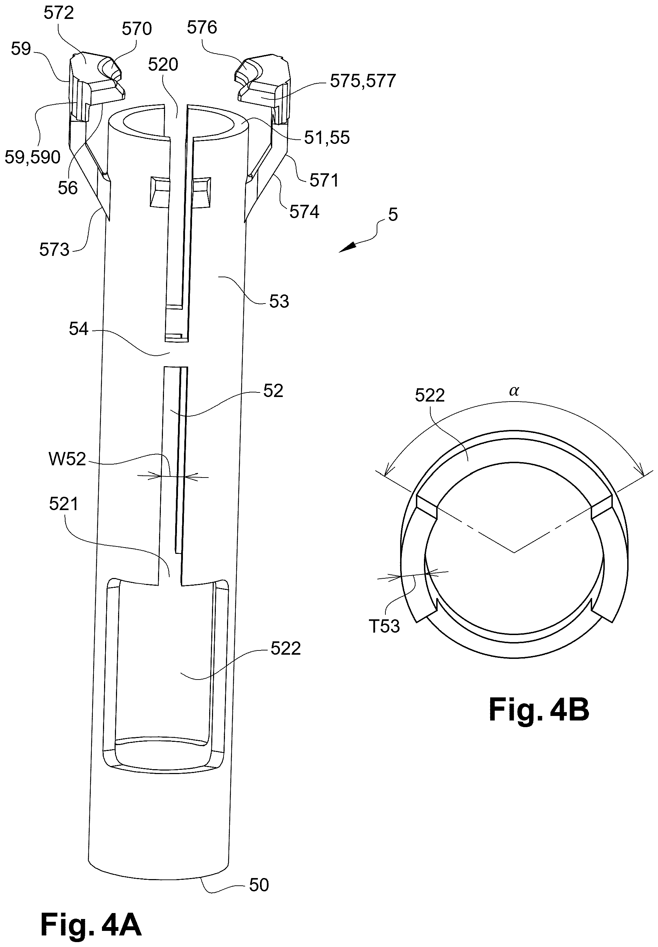

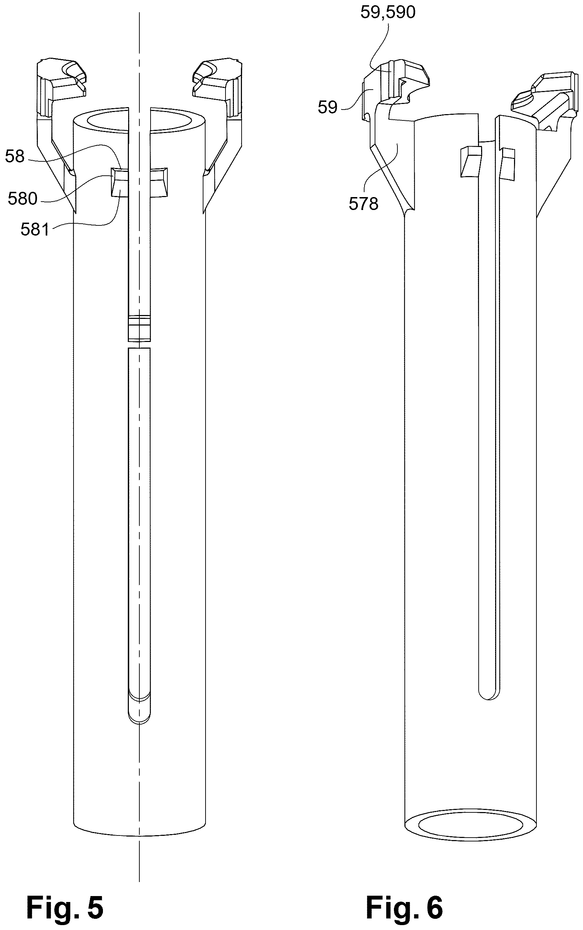

With reference to Figure 4A, 4B, 5 and 6 is shown the spacer 5. The spacer 5 is arranged within the body 2, and is configured for receiving the medical container 6. The spacer 5 is in the form of a cylindrical sleeve including an opened distal end 50 and an opened proximal end 51. The spacer 5 has an axial slot 52 for receiving the emboss 62 of the medical container 6, such that the medical container 6 can be inserted in the spacer 5 and so that the emboss 62 of the medical container 6 can slide in the axial slot 52 when the medical container 6 is positioned within the spacer 5. The axial slot 52 may have an opened proximal end 520 at the proximal end 51 of the spacer 5, and an opened distal end 521, Figure 4A, which opens into a window 522 arranged through a lateral wall 53 of the spacer 5. The window 522 is larger than the axial slot 52, and the length of the window 522 is greater than the length of the emboss 62 such that the window 522 allows for rotation of the medical container 6 relative to the spacer 5. As illustrated in Figure 4B, the window 522 may define for instance a 120° angle α. In other embodiments, as shown in Figures 5 and 6, the axial slot 52 may have a closed distal end and the spacer 5 does not have any window 522, so that the medical container 6 is rotationally fixed with respect to the spacer 5. The width W52 of the axial slot 52 may be equal to or slightly greater than a width W62 of the emboss 62. The spacer 5 may include one or more axial slots 52, for example two diametrically opposite axial slots 52, and one or more windows 522, such as two diametrically opposite windows 522.

The thickness T53 of the lateral wall 53 of the spacer 5 is equal to or greater than the height H62 of the emboss 62 so that the emboss 62 does not protrude over the spacer 5. That is, the emboss 62 does not radially outwardly extend outside the spacer 5. As a result, the emboss 62 creates no interference with the body 2. The thickness T53 of the lateral wall 53 of the spacer 5 is actually designed to compensate the radial clearance that would exist between the barrel 60 of the medical container 6 and the body 2 if the spacer 5 was not there. Thus, the medical container 6 cannot move within the body 2, is firmly maintained within the body 2, well centered and aligned with respect to the longitudinal axis A. The spacer 5 is layered between the medical container 6 and the body 2. An outer surface of the lateral wall 53 of the spacer 5 may radially abut against an inner surface of the body 2 while. An inner surface of the lateral wall 53 of the spacer 5 may radially abut against an outer surface of the barrel 60 of the medical container 6.

It is contemplated that the length of the spacer 5 may distally extend outside the body 2 to define a predetermined pricking depth, the distal end 50 of the spacer 5 abutting against the injection site during use of the injection device 10. In such a case, the spacer 5 extends over a portion of the needle shield 69, but does not completely extend over the needle shield 69: at least a distal portion of the needle shield 69 needs to remain accessible to the user such that the user can grasp and pull the needle shield 69 off the medical container 6.

As illustrated in Figure 4A or 5, the spacer 5 may include breakable bridges 54 tangentially extending in the axial slot 52 for connecting two opposite sides of the axial slot 52. The breakable bridges 54 may be closer to the proximal end 520 of the axial slot 52 than to the distal end 521 of the axial slot 52. The breakable bridges 54 and the lateral wall 53 of the spacer 5 to which they are connected may be made of a single piece. The breakable bridges 54 are designed to break when the spacer 5 radially outwardly deform when the medical container 6 is assembled to the spacer 5. Before this assembly, the breakable bridges 54 allow for providing some rigitdity to the spacer 5, thus allowing better handling of the spacer 5. As illustrated in Figure 6, the spacer 5 may be devoid of beakable bridges.

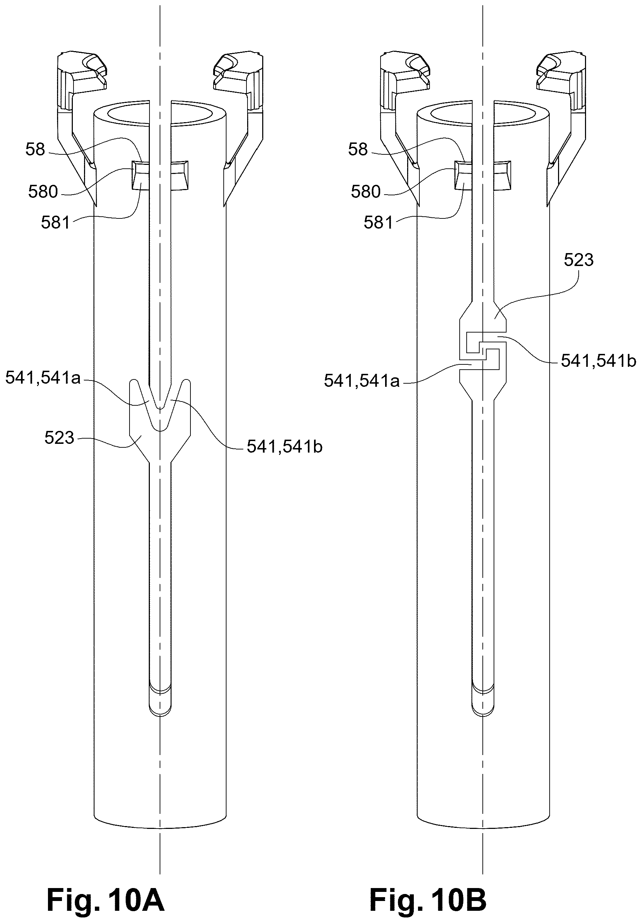

With reference to Figures 10A and 10B, instead of a breakable bridge 54, the spacer 5 may include one or more deformable ties 541 configured for limiting radial outward deformation of the two hemicylindrical halves of the spacer 5, without breaking. The ties 541 are configured not to break when the spacer 5 radially outwardly deforms nor when the emboss 62 passes over these ties 541. The ties 541 may be lodged in a widened section 523 of the axial slot 52.

As illustrated in Figure 10A, the tie 541 includes two connected portions 541a, 541b, and thus connects the two sides of the axial slot 52. The tie 541 may be V-shaped, apex extending in the distal direction to ease passage of the emboss 62. The tie 541 may further be located at one end, such as a proximal end of the widened section 523. The tie 541 and the spacer 5 may be made of a single piece.

In the embodiment illustrated in Figure 10B, the tie 541 includes two separate interlocked portions 541a, 542b, each extending from an opposite side of the axial slot 52. Although they are separated, the two portions 541a, 542b of the tie 541 are configured for tangentially abutting against each other to limit deformation of the spacer 5. As shown in Figure 10B, the two portions 541a, 542b may be L-shaped. The interlocked L-shaped portions 541a, 542b of the tie 541 may here be located between the two ends of the widened section 523, substantially at the middle of the widened section 523. The two portions 541a, 541b of the tie 541 and the spacer 5 may be made of a single piece.

The spacer 5 may include a proximal seat 55 for axially abutting against a distal side of the radial flange 61 of the medical container 6, thereby stopping insertion of the medical container 6 and axially blocking the medical container 6 with respect to the spacer 5. The proximal seat 55 may arranged at the proximal end of the spacer 5 and may specifically be defined by the rim delimiting the opened proximal end of the spacer 5.

Opposite the proximal seat 55, the spacer 5 includes a distal blocking surface 56, which may be provided on snap-fit elements 57, for axially abutting against a proximal side of the radial flange 61 of the medical container 6, thereby preventing withdrawal of the medical container 6 from the spacer 5. The medical container 6 is thus axially fixed to the spacer 5. This for instance helps prevent axial movement of the medical container 6 during drop tests. The distal blocking surface 56 may extend in a circumferential direction, over the circular portions 611 of the cut flange 61 of the medical container 6. The distal blocking surface 56 may be located at a distal side of a radially inward hook 570 extending at a free proximal end 572 of a resilient arm 571 whose distal end 573 is connected to the lateral wall 53 of the spacer 5, near or at the proximal end of the spacer 5. The hook 570 has two circumferentially protruding ears 575 at both sides of the proximal end of the resilient arm 571. The hook 570 includes a camming surface 576 configured for abutting against the flange 61 of the medical container 6, thereby causing outward deformation of the snap-fit elements 57 so that the hook 570 can pass over the flange 61 of the medical container 6 when the medical container 6 is assembled to the spacer 5. An axial distance between the proximal seat 55 and the distal blocking surface 56 may be equal to or greater than the axial thickness of the radial flange 61 of the medical container 6. The distal blocking surface 56 and the proximal seat 55 of the spacer 5 form first fasteners 55, 56 for axially securing the medical container 6 with respect to the spacer 5.

The resilient arm 571 may include a stop surface 574, which may here extend obliquely with respect to the longitudinal axis A, configured for abutting against a corresponding stop surface 26 of the body 2. The stop surfaces 26, 574 may be complementarily shaped, the stop surface 26 of the body 2 being also inclined with respect to the longitudinal axis A.

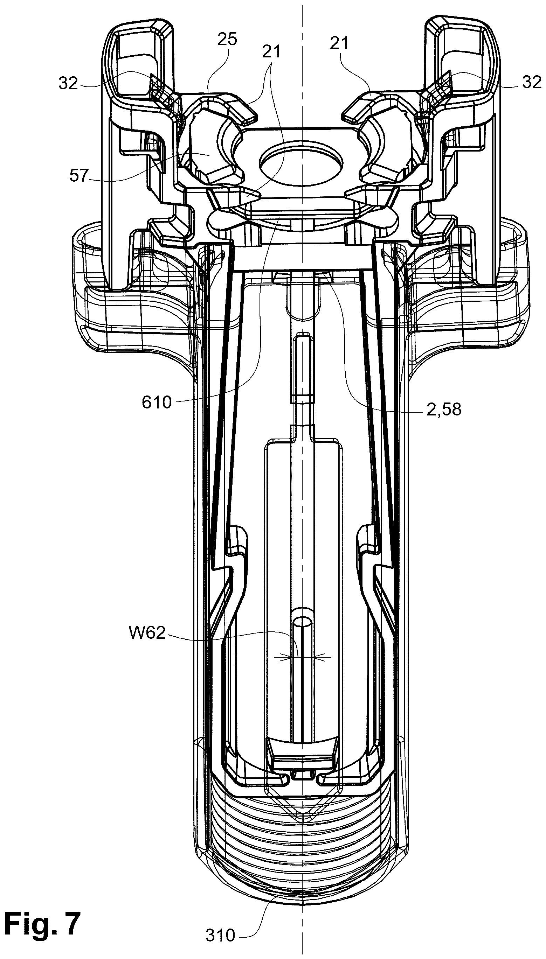

As visible in Figure 7, the snap-fit element 57 may include a radial abutment 59 arranged on an outer surface of the hook 570 for radially abutting against the body 2 such that the snap-fit element 57 cannot flex back once the spacer 5 is inserted within the body 2. Accordingly, the distal blocking surface 56 of the spacer 5 cannot be shifted away from the axial path of the radial flange 61 of the medical container 6. In the illustrated embodiment, the radial abutment 59 may be arranged at an outer surface of the hook 570, proximal to the proximal end of the reasilient arm 571, and/or may be arranged at an outer surface of the tangentially protruding ears 575 in the form of axial ribs 590 protruding from the outer surface of the protruding ears 575.

As illustrated in Figure 7, the hook 570 extends between two orthoradial abutments 25 of the body 2 and may include two lateral sides 577 for orthoradially abutting against the body 2. The orthoradial abutments 25 may be delimited by the blocking members 21 of the body 2. That is, the snap-fit elements 57 of the spacer 5 extend between two blocking members 21 of the body 2. Therefore, the spacer 5 is rotationally fixed with respect to the body 2.

With reference to Figure 6, instead of resilient arms 571, the snap-fit elements 57 may be rigidly connected to the spacer 5 by a connecting plate 578. In this embodiment, the snap-fit elements 57 are rigid instead of being deformable. The whole spacer 5 is deformale due to the two diametrically opposite axial slots 52, devoid of any breakable bridge 54 or tie 541, which run from the proximal end 51 of the spacer 5 and which split the spacer 5 in two flexible half-tubes.

In an embodiment, not shown, the snap-fit elements 57 may include a proximal blocking surface 58 configured for axially abutting against a distal abutment of the blocking members 21 of the body 2. The proximal blocking surface 58 may be a circumferential leg extending from a lateral side of the hook 570 of the snap-fit element 57 and beneath the blocking members 21 of the body 2, such that proximal movement of the spacer 5 with respect to the body 2 is prevented.

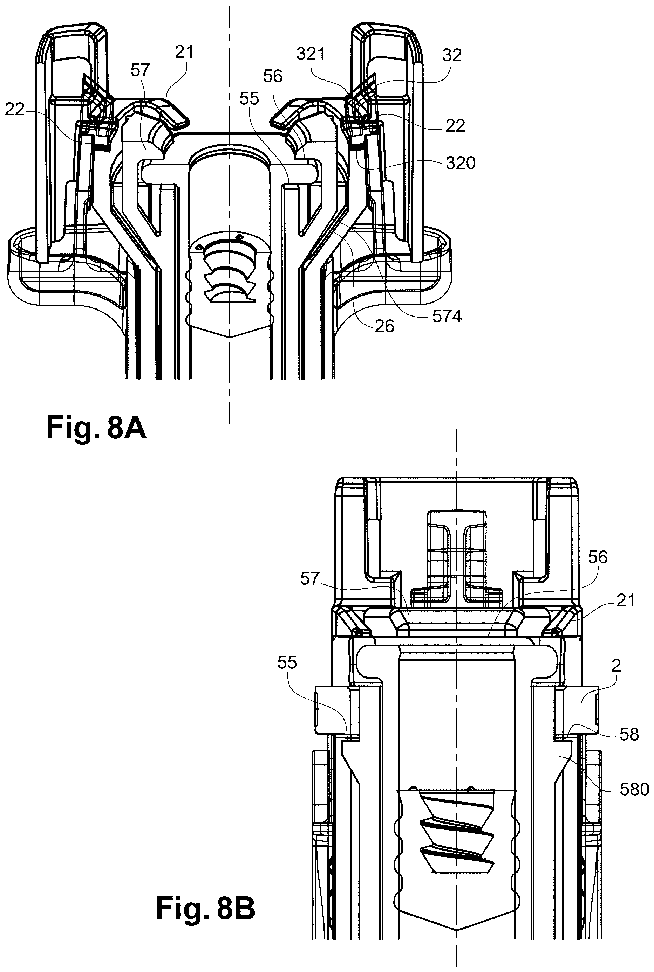

Alternatively, as illustrated for example in Figures 4A and 8B, the spacer 5 includes a proximal blocking surface 58 configured for axially abutting against the body 2, such that proximal movement of the spacer 5 with respect to the body 2 is prevented. Specifically, the proximal blocking surface 58 may here be arranged at a proximal side of a radial protrusion 580 which radially outwardly protrude from an outer surface of the lateral wall 53 of the spacer 5. The radial protrusion 580 may include an opposite distal side in the form of a ramp surface 581 for easing insertion of the spacer 5 into the body 2. In the illustrated embodiment, the spacer 5 may include one or more radial protrusions 580, for instance two radial protrusions 580 arranged at both sides of the axial slot 52. The distance between the two protrusions 580 here corresponds to the width W52 of the axial slot 52. The radial protrusions 580 may be located near the proximal end 51 of the spacer 5, or at least closer to the proximal end 51 than to the distal end 50 of the spacer 5 to avoid interference with the needle guard 3. Specifically, as visible in Figure 5, the radial protrusions 580 may extend at a similar axial location as the distal end of the snap-fit elements 57. The snap-fit elements 57 and the radial protrusions 580 may be angularly separated by a 90° angle. The radial protrusions 580 and the lateral wall 53 of the spacer 5 may be made of a single piece. The radial protrusions 580 may be proximal to the needle guard 3 such that distal movement of the needle guard 3 is not hindered by the radial protrusions 580. As visible in Figure 8B, the radial protrusions 580 may be radially distant from the needle guard 3 to avoid interaction with the needle guard 3. The proximal blocking surface 58 and the stop surface 574 of the spacer 5 may form second fasteners 58, 574 for axially securing the spacer 5 with respect to the body 2.

The safety device 1 of the invention thus allows for safely using a cut-flange 61 by-pass syringe.

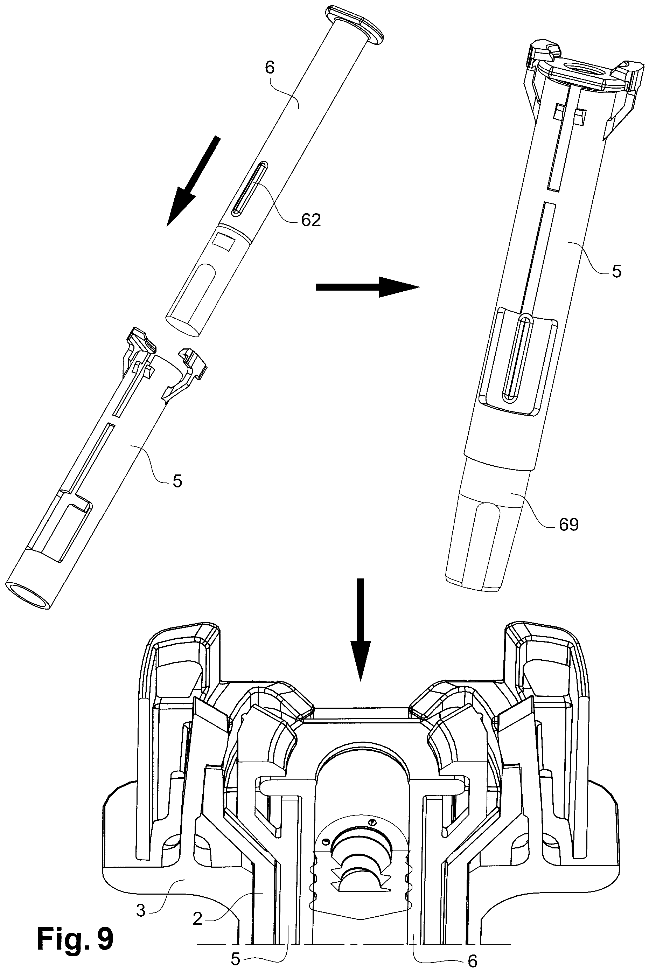

The method for assembling the injection device 10 of the invention is described with reference to Figure 9.

The medical container 6 is first positioned within the spacer 5. The relative angular position between the medical container 6 and the spacer 5 is set by the one or more axial slots 52. If the spacer 5 includes two diametrically opposite axial slots 52, as illustrated in the Figures, then there are two possible angular positions of the medical container 6 with respect to the spacer 5. The angular positions may be designed such that the snap-fit elements 57 of the spacer 5 abut againt the circular portions 611 of the radial flage of the medical container 6.

The emboss 62 of the medical container 6 is aligned with the axial slot 52 such that the emboss 62 can enter and slide along the axial slot 52. Insertion of the medical container 6 stops when the radial flange 61 of the medical container 6 distally abuts against the proximal seat 55 of the spacer 5. At this stage, the snap-fit elements 57 flex back to their initial position after having been radially outwardly deformed by the radial flange 61 of the medical container 6, such that the distal blocking surface 56 of the spacer 5 prevents the medical container 6 from moving back in the proximal direction. The medical container 6 may be axially fixed to the spacer 5. If the spacer 5 has a window 522, the medical container 6 may however have a limited rotation with respect to the spacer 5.

The spacer 5 and the medical container 6 are then introduced within the body 2, until the stop surfaces of the spacer 5 and of the body 2 abut against each other, thus preventing further distal movement of the spacer 5 with respect to the body 2. Meanwhile, the proximal blocking surface 58 of the spacer 5 proximally has come into abutment against the body 2, thereby preventing proximal movement of the spacer 5 relative to the body 2 and thus preventing removal of the spacer 5 from the body 2, for instance during drop tests. The snap-fit elements 57 get lodged between the blocking members 21 of the body 2, so that the spacer 5 cannot rotate with respect to the body 2. The injection device 10 is assembled and ready to be used.

It is readily understandable from the above description that the safety device 1 of the invention is suitable for by-pass syringes. The injection device 10 that includes this reusable safety device 1 allows for performing a manual injection operation, while benefitting from an automatic activation of the safety device 1. It is to be understood that the present invention is not limited to the embodiments described above and illustrated in the drawings; rather, the skilled person will recognize that many changes and modifications may be made within the scope of the appended claims.

## Claims

1. Safety device (1) configured for protecting a user against needle stick injuries, the safety device (1) including:
a body (2) extending along a longitudinal axis A and configured for receiving a medical container (6) provided with an injection needle (66), a plunger rod (68), and a barrel (60), the barrel (60) including an emboss (62),
a needle guard (3) axially movable with respect to the body (2) between an initial position and a safety position distally located with respect to the initial position,
biasing means for moving the needle guard (3) from the initial position to the safety position,
a retainer (32), movable between a blocking position in which the retainer (32) blocks the needle guard (3) in the initial position against the action of the biasing means, and a release position in which the retainer (32) no longer prevents the biasing means from moving the needle guard (3) to the safety position, movement of the retainer (32) from the blocking position to the release position being caused by the plunger rod (68) at the end of injection, wherein the safety device (1) further includes
a spacer (5) arranged between the body (2) and the medical container (6) for compensating a gap the body (2) and the medical container (6),
an axial slot (52) extending along the spacer (5) for receiving the emboss (62) of the medical container (6),
first fasteners (55, 56) for axially fixing the medical container (6) to the spacer (5), and
second fasteners (58, 574) for axially fixing the spacer (5) to the body (2).

2. Safety device (1) according to the preceding claim, wherein the first fasteners (55, 56) include snap-fit elements (57) configured for blocking axial movement of the medical container (6) relative to the spacer (5) in a proximal direction.

3. Safety device (1) according to the preceding claim, wherein the snap-fit elements (57) are wholly positioned distal to a transversal plane including a distal blocking surface (56) of the retainers (32).

4. Safety device (1) according to the preceding claim 2 or 3, wherein the snap-fit elements (57) are configured for axially abutting against a proximal side of a flange (61) of the medical container (6).

5. Safety device (1) according to the preceding claims 2 to 4, wherein the snap-fit elements (57) include a radial abutment (59) surface configured for radially abutting against an inner surface of the body (2) to prevent radial outward movement of the snap-fit elements (57) when the spacer (5) is arranged within the body (2).

6. Safety device (1) according to the preceding claim, wherein the radial abutment surface (59) is delimited by one or more axial ribs (590) protruding from an outer surface of the snap-fit elements (57).

7. Safety device (1) according to any one of the preceding claims, wherein a distal end of the axial slot (52) opens on a window (522) configured for receiving the emboss (62) of the medical container (6) and for allowing rotation of the medical container (6) with respect to the spacer (5).

8. Safety device (1) according to any one of the preceding claims, wherein the second fasteners (58, 574) include a proximal blocking surface (58) arranged on a radial outward protrusion (580) for axially abutting against the body (2) to prevent removal of the spacer (5) from the body (2).

9. Safety device (1) according to the preceding claim, wherein the radial outward protrusion (580) is radially and/or axially distant from the needle guard (3) to avoid interference between the spacer (5) and the needle guard (3).

10. Safety device (1) according to any one of the preceding claims, wherein the spacer (5) includes one or more breakable bridges (54) connecting two opposite sides of the axial slot (52) of the spacer (5) before insertion of the medical container (6) within the spacer (5), the breakable bridges (54) being configured to break upon radial outward deformation of the spacer (5) when the medical container (6) is inserted within the spacer (5).

11. Injection device (10) including a medical container (6) and a safety device (1) according to any one of the preceding claims 1 to 10.

12. Injection device (10) according to the preceding claim, wherein the medical container (6) is a by-pass syringe.

13. Injection device (10) according to any one of claim 11 or 12, wherein the medical container (6) has a cut flange (61).

14. Method for assembling an injection device (10) according to any of claims 11-13, the method comprises a step of assembling the safety device (1) without the spacer (5) to obtain a first sub-assembly (2, 3, 4); a step of assembling the medical container (6) to the spacer (5) to obtain a second sub-assembly (5, 6); and a step of inserting the second sub-assembly (5, 6) into the first sub-assembly (2, 3, 4).
